(19) Europäisches Patentamt
European Patent Office
Office européen des brevets

(11) Publication number: **0 158 709**
**B1**

(12) **EUROPEAN PATENT SPECIFICATION**

(45) Date of publication of patent specification: **29.07.87**

(21) Application number: **84112783.0**

(22) Date of filing: **09.03.82**

(60) Publication number of the earlier application in accordance with Art. 76 EPC: **0 073 837**

(51) Int. Cl.[4]: **C 07 C 49/647,** C 07 C 65/03, C 07 C 37/18, C 07 C 45/00, C 07 C 51/093

# (54) A process for producing a para-substituted phenol derivative.

(30) Priority: **09.03.81 JP 32542/81**
**11.08.81 JP 125558/81**
**13.08.81 JP 127077/81**
**25.09.81 JP 151571/81**
**26.09.81 JP 152524/81**

(43) Date of publication of application:
**23.10.85 Bulletin 85/43**

(45) Publication of the grant of the patent:
**29.07.87 Bulletin 87/31**

(84) Designated Contracting States:
**CH DE FR GB LI**

(56) References cited:
**GB-A- 864 720**

**MAKROMOL. CHEM., RAPID COMMUN., vol. 2, 1981, pages 601-603; MAKOTO KOMIYAMA et al.: *"Selective catalysis of cyclodextrin in the synthesis of 4-allyl-2,4,6-trimethyl-2,5-cyclohexadienone"***

**The file contains technical information submitted after the application was filed and not included in this specification**

(73) Proprietor: **Asahi Kasei Kogyo Kabushiki Kaisha**
**2-6, Dojimahama 1-chome Kita-ku**
**Osaka-shi Osaka 530 (JP)**

(72) Inventor: **Hirai, Hidefumi**
**14-10, Yutenji 1-chome**
**Meguro-ku Tokyo 153 (JP)**
Inventor: **Komiyama, Makoto**
**16-1-508, Kosuge 4-chome**
**Katsushika-ku Tokyo 124 (JP)**

(74) Representative: **Schübel-Hopf, Ursula et al**
**Strehl, Schübel-Hopf, Schulz Patentanwälte**
**Widenmayerstrasse 17**
**D-8000 München 22 (DE)**

(56) References cited:
**THE JOURNAL OF ORGANIC CHEMISTRY, vol. 30, no. 11, November 1965, pages 3895-3897; B.MILLER et al.: *"Effects of solvent and of the alkylating agent upon alkylation of the 2,6-di-t-butyl-4-methylphenoxide anion"***

**EP 0 158 709 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for producing a para-substituted phenol derivative with high selectivity. More particularly, the present invention is concerned with a process for producing a para-substituted phenol derivative which comprises reacting a phenol compound with an organic halide selected from the group consisting of a carbon tetrahalide and a substituted or unsubstituted allyl halide in the presence of an alkali metal hydroxide, using a cyclodextrin as a catalyst.

As to the production of para-hydroxybenzoic acid which, nowadays, is of increasing importance as a raw material for heat resistant polymers, agricultural chemicals and pharmaceuticals, known is the Kolbe-Schmitt reaction in which para-hydroxybenzoic acid is synthesized by treating phenol with potassium hydroxide and potassium carbonate, followed by heating together with carbon dioxide under elevated pressure. The reaction, however, has disadvantages that a costly pressure resistant apparatus is required because of high pressure applied during the reaction, and that much energy is required for the achievement of an absolute anhydrous condition which is indispensable to the reaction. Also known is another process in which phenol is reacted with a carbon tetrachloride in the presence of an alkali metal hydroxide to prepare para-hydroxybenzoic acid. In the process, however, the selectivity for the formation of para-hydroxybenzoic acid is 57%, and the reaction gives a large amount of salicylic acid as a by-product. Therefore, the process also requires not only large amounts of raw materials but also an operation for separation.

2,5-Cyclohexadienone derivatives having an allyl group at the 4-position are also highly reactive due to the conjugation of two C—C double bonds and a carbonyl group. In addition, they have an allyl group at such a position that an intramolecular ring-forming reaction is readily caused to occur. Accordingly, the derivatives are valuable compounds as starting materials for preparing physiologically active substances and other useful substances 2,5-Cyclohexadienone derivatives having an allyl group at the 4-position have conventionally been prepared in a process comprising two steps, namely, the first step in which a 1:1 mixture of sodium methoxide and a para-substituted phenol is reacted with an allyl halide in an aromatic solvent to produce a 2,4-cyclohexadienone derivative which is allyl-substituted at the 6-position, and the second step in which the product in the first step is then reacted in a methanol-hydrochloric acid mixture to allow the allyl group to transfer to the 4-position. The process has disadvantages that the 2,4-cyclohexadienone derivative which is a reaction product of the first step is difficult to separate and purify, and that the reactions involved in the process require large amounts of organic solvents.

As described above, any of the conventional processes for introducing a substituent derived from an organic halide selected from a carbon tetrachloride and a substituted or unsubstituted allyl halide to the para-position of phenols are unsatisfactory from a practical point of view because of extremely low selectivity or because of necessity of two reaction steps accomplished by other difficulties during the reaction.

The present inventors have made extensive and intensive studies to develop a process in which a substituent derived from an organic halide selected from a carbon tetrahalide and a substituted or unsubstituted allyl halide (hereinafter often referred to simply as "organic halide") is introduced to the 4-position of phenols with high selectivity to give intermediates for the production of various useful substances as mentioned above. As a result, it has been found that, when a phenol compound is reacted with an organic halide in the presence of cyclodextrin under an alkaline condition, a substituent group derived from said organic halide is introduced to the 4-position of the phenol compound with high selectivity, and therefore the intended para-substituted phenol derivative can be obtained in high yield. Based on such a novel finding the present invention has been made.

Accordingly, it is an object of the present invention to provide a process for producing a para-substituted phenol derivative with high selectivity.

According to the present invention, there is provided a process for producing a para-substituted phenol derivative which comprises reacting a phenol compound represented by the formula (I)

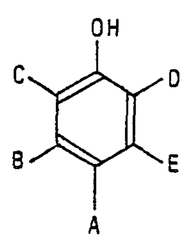

(I)

wherein A, B, C, D, and E each independently stand for hydrogen, a hydroxyl group, a substituted or unsubstituted alkyl group, a substituted or unsubstituted allyl group, a substituted or unsubstituted alkoxyl group or a substituted or unsubstituted aryl group, provided that A does not stand for a hydroxyl group and that when two or more A, B, C, D, and E each independently stand for a substituted or unsubstituted alkyl group or a substituted or unsubstituted alkoxy group, they have their respective free terminal ends or at least one of them is bonded to another group selected from said alkyl and alkoxyl groups to form a ring, with an organic halide in the presence of an alkali metal hydroxide, using as a catalyst a modified or

unmodified cyclodextrin, characterized in that said organic halide is selected from the group consisting of a carbon tetrahalide and a substituted or unsubstituted allyl halide, thereby to introduce a substituent group derived from the organic halide selected from the group consisting of a carbon tetrahalide and a substituted or unsubstituted allyl halide to the para-position of the phenol compound.

By the term "phenol compound" as used herein is meant phenol (hydroxybenzene) or its derivative which is defined by the above-mentioned formula (I). The substituted or unsubstituted alkyl group, the substituted or unsubstituted allyl group, the substituted or unsubstituted alkoxyl group and the substituted or unsubstituted aryl group each may preferably have carbon atoms of not more than 6 with respect to the substituents B, C, D and E, and each may preferably have carbon atoms of not more than 12 with respect to the substituent A.

As the substituent to be introduced to the alkyl group, allyl group, alkoxyl group and aryl group, there can be mentioned an alkyl group, a halogen atom and others without specific restriction. However, too large a group is undesired.

The organic halide which is one of the reactants to be used in the process of the present invention is selected from the group consisting of a carbon tetrahalide and a substituted or unsubstituted allyl halide. The substituted or unsubstituted allyl halide may preferably have carbon atoms of not more than 12. As such an allyl halide, the chloride and bromide are especially preferred. The organic halide may be used in an equimolar amount to the amount of phenol compound, but usually in an amount which is twice to 20 times the amount of the phenol compound, because the organic halide tends to decompose in an aqueous medium used as a reaction solvent, about which aqueous medium a description will be given later.

The alkali metal hydroxide to be used in the process of the present invention may preferably be sodium hydroxide or potassium hydroxide. The alkali metal hydroxide may be used in a stoichiometrical amount relative to the phenol compound. Usually, however, 1 to 15 times the stoichiometrical amount of the alkali metal hydroxide may be used taking into consideration of the rate of reaction and the like.

The reaction according to the process of the present invention is usually carried out in a reaction medium. As the the reaction medium, there is employed an aqueous solvent, preferably water, because of the requirement that the reaction medium be capable of dissolving the alkali metal hydroxide therein. There may also be used, as the reaction medium, a mixture of water with a small amount of an organic solvent which is soluble in water and can be present stably under the reaction conditions. Examples of such an organic solvent include methanol, ethanol, acetone, dimethoxyethane and the like. The concentration of the alkali metal hydroxide in the reaction solvent may be in the range of 5 to 20% by weight, preferably 10 to 15% by weight.

In the process according to the present invention, a better result in the reaction can be achieved by controlling the molar ratio of a modified or unmodified cyclodextrin (hereinafter often referred to simply as "cyclodextrin") to an organic halide selected from the group consisting of a carbon tetrahalide and a substituted or unsubstituted allyl halide in the reaction system. To effect the reaction economically and advantageously, the molar ratio of the cyclodextrin to the organic halide may usually be 0.1 to 20, preferably 0.2 to 15, more preferably 0.5 to 10. When a large amount of the organic halide is present in the reaction system, a large amount of cyclodextrin is required to obtain a better result in the reaction. On the other hand, when the organic halide is dropwise added to a solution containing a phenol compound, an alkali hydroxide and cyclodextrin so that the molar ratio of the cyclodextrin to the organic halide is maintained in the range of from 0.1 to 20, preferably 0.2 to 15, more preferably 0.5 to 10, a better result in the reaction can be attained with a small amount of cyclodextrin, leading to economical advantages.

The above-mentioned modified or unmodified cyclodextrin acts as a catalyst in the reaction of the process according to the present invention, and any of modified or unmodified α-, β- and γ-cyclodextrins may be used. Usually, satisfactory results can be obtained by the use of unmodified α-, β- or γ-cyclodextrin. However, a more improved yield and selectivity in the reaction are achieved by the use of a modified α-, β- or γ-cyclodextrin of which the primary hydroxyl groups, for example, are all or partly substituted with a group which is stable under alkaline conditions, such as a N-methylformamido group. The abovementioned modified cyclodextrin may be prepared according to the method described in J. Amer. Chem. Soc., *102,* 762 (1980).

The reaction temperature is not critical, and the reaction may be carried out at room temperature or higher temperature, preferably at a temperature of from 50°C to 80°C.

The reaction time is also not critical, and may be suitably determined according to the amounts of reactants, reaction temperature, manner of addition of reactants and the like.

The reaction pressure is also not restricted, and the reaction is usually carried out at atmospheric pressure from a viewpoint of ease in operation.

By the reaction of the phenol compound with the organic halide according to the process of the present invention, there is produced a para-substituted phenol derivative of the kind varied depending on the kinds of the phenol compound and the organic halide, as described later.

From phenol compounds of the formula (I) in which A is a hydrogan atom, para-hydroxybenzoic acids, or para-allyl phenols are obtained.

From phenol compounds of the formula (I) in which A is other substituent than hydrogen, namely, a substituted or unsubstituted alkyl group, a substituted or unsubstituted allyl group, substituted or unsubstituted alkoxyl group or substituted or unsubstituted aryl group, there is obtained 4-allyl-2,5-cyclohexadienone derivatives. Illustratively stated, when A in the formula (I) is hydrogen, there is obtained a para-substituted phenol derivative represented by the formula (II)

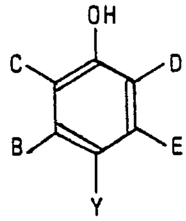

(II)

wherein B, C, D and E are as defined above and Y stands for a substituted or unsubstituted allyl group or a carboxyl group, whereas when A in the formula (I) is a substituted or unsubstituted alkyl group, a substituted or unsubstituted allyl group, a substituted or unsubstituted alkoxyl group or a substituted or unsubstituted aryl group, there is obtained a para-substituted phenol derivative represented by the formula (III)

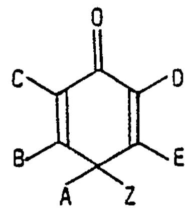

(III)

wherein A, B, C, D and E are as defined above, provided that A does not stand for hydrogen, and Z stands for a substituted or unsubstituted alkyl group.

As is apparent from the foregoing, according to the process of the present invention, a variety of useful phenol derivatives having a substituent introduced to the para-position thereof are produced from phenols with high selectivity. Therefore, not only can be reduced the required amounts of phenols as raw materials but also the purification process can be extremely simplified, thus enabling the production process of the desired products to be economic.

The present invention will be illustrated in more detail with reference to the following Examples. Unless otherwise specified, reactions were carried out at atmospheric pressure in Examples and Comparative Examples as will be described hereinafter.

Example 1

In 20 ml of an aqueous 20% sodium hydroxide solution were dissolved 1.5 g of phenol (first class grade reagent, manufactured and sold by Koso Chemical Co., Ltd., Japan) and 1.5 g of β-cyclodextrin (special grade reagent, manufactured and sold by Nakarai Chemical Ltd., Japan). To the resulting solution was added 3 ml of carbon tetrachloride (first class grade reagent, manufactured and sold by Tokyo Kasei Co., Ltd., Japan) and 0.1 g of copper powder (first class grade reagent, manufactured and sold by Yoneyama Yakuhin Kogyo Co., Ltd., Japan). The resulting mixture was allowed to react at 80°C for 10 hours under reflux by the use of a reflux condenser while agitating by means of a magnetic stirrer. After completion of the reaction, the solution was acidified with hydrochloric acid, and subjected to extractions each with 50 ml of ethyl ether 3 times. The ethyl ether layer was washed with water, and then dried, thereby to obtain 2.1 g of a product. The infrared spectrum of the product thus obtained was in agreement with that of para-hydroxybenzoic acid (special grade reagent, Tokyo Kasei Co., Ltd., Japan). Further, the product was treated with chloroform and, as a result, it was found that the product did no contain any chloroform-soluble matter any more. Since both phenol if any remaining unreacted and salicylaldehyde if any formed as a side reaction product are readily soluble, in chloroform, the yield of the intended product was 95% and the selectivity was 100%.

Comparative Example 1

Each reagent used herein was of the same grade as in Example 1. In 20 ml of an aqueous 20% sodium hydroxide solution was dissolved 1.5 g of phenol. To the resulting solution, 3 ml of carbon tetrachloride and 0.1 g of copper powder were added. The resulting mixture was allowed to react at 80°C for 10 hours under reflux by the use of a reflux condenser while agitating by means of a magnetic stirrer. After completion of the reaction, the reaction mixture was acidified with hydrochloric acid, and subjected to extractions each with 50 ml of ethyl ether 3 times. The ethyl ether layer was washed with water, and then dried, thereby to obtain 2.1 g of a product. The product thus obtained was treated with chloroform to give 1.2 g of a chloroform-insoluble matter and 0.9 g of a chloroform-soluble matter. The infrared spectrum of the chloroform-insoluble matter was in agreement with that of para-hydroxybenzoic acid. On the other hand, the infrared spectrum of the chloroform-soluble matter was in agreement with that of salicylic acid

(special grade reagent, manufactured and sold by Koso Chemical Co., Ltd., Japan). Namely, the yield of the intended product was 55% and the selectivity was 57%.

Example 2

In 50 ml of an aqueous 1% sodium hydroxide solution were dissolved 0.20 g of 2,4,6-trimethylphenol (special grade reagent, manufactured and sold by Tokyo Kasei Co., Ltd., Japan) and 7.5 g of α-cyclodextrin (special grade reagent, manufactured and sold by Nakarai Chemical Ltd., Japan). While dropwise adding 0.9 g of allyl bromide (special grade reagent, manufactured and sold by Tokyo Kasei Co., Ltd., Japan) to the resulting solution at room temperature, the reaction was allowed to proceed for 24 hours. After completion of the reaction, the reaction mixture was subjected to extractions each with 50 ml of ether 5 times. The ether layer was dried to obtain 0.18 g of a product. The $^1H$—NMR measurement showed that the product was a mixture of 53% of 2,4,6-trimethyl-4-allyl-2,5-cyclohexadienone, 26% of 2,4,6-trimethyl-6-allyl-2,4-cyclohexadienone and 21% of the 2,4,6-trimethylphenyl allyl ether. Namely, the yield of the intended product and the selectivity were 37% and 53%, respectively.

Comparative Example 2

In 50 ml of an aqueous 1% sodium hydroxide solution was dissolved 0.20 g of 2,4,6-trimethylphenol (special grade reagent, manufactured and sold by Tokyo Kasei Co., Ltd., Japan). While dropwise adding 0.9 g of allyl bromide (special grade reagent, manufactured and sold by Tokyo Kasei Co., Ltd., Japan) to the resulting solution at room temperature, the reaction was allowed to proceed for 24 hours. After completion of the reaction, the reaction mixture was subjected to extractions each with 50 ml of ethyl ether 5 times. The ethyl ether layer was dried to obtain 0.19 g of a product. The $^1H$—NMR measurement showed that the product was a mixture of 25% of 2,4,6-trimethyl-4-allyl-2,5-cyclohexadienone, 50% of 2,4,6-trimethyl-6-allyl-2,4-cyclohexadienone and 25% of 2,4,6-trimethylphenyl allyl ether. Namely, the yield of the intended product and the selectivity were 18% and 25%, respectively.

Example 3

Substantially the same procedures as in Example 2 were repeated except that 7.5 g of β-cyclodextrin (special grade reagent, manufactured and sold by Nakarai Chemical Ltd., Japan) was used instead of 7.5 g of α-cyclodextrin. There was obtained 0.20 g of a product. The $^1H$—NMR measurement showed that the product was a mixture of 41% of 2,4,6-trimethyl-4-allyl-2,5-cyclohexadienone, 32% of 2,4,6-trimethyl-6-allyl-2,4-cyclohexadienone and 27% of 2,4,6-trimethylphenyl allyl ether. Namely, the yield of the intended product and selectivity were 32% and 41%, respectively.

Example 4

In accordance with the method as described in J. Amer. Chem. Soc., *102* 762 (1980), all of the primary hydroxyl groups of α-cyclodextrin were substituted by N-methylformamido groups. In 50 ml of an aqueous 1% sodium hydroxide solution were dissolved 8 g of the above-obtained modified cyclodextrin and 0.20 g of 2,4,6-trimethylphenol. While dropwise adding 0.9 g of allyl bromide (special grade reagent, manufactured and sold by Tokyo Kasei Co., Ltd., Japan) to the resulting solution at room temperature, the reaction was allowed to proceed for 24 hours. After completion of the reaction, the reaction mixture was subjected to extractions each with 50 ml of ether 5 times. The ethyl ether layer was dried to obtain 0.22 g of a product. The $^1H$—NMR measurement showed that the product was entirely 2,4,6-trimethyl-4-allyl-2,5-cyclohexadienone. Namely, the yield of the product and selectivity were 85% and 100%, respectively.

**Claims**

1. A process for producing a para-substituted phenol derivative which comprises reacting a phenol compound represented by the formula (I)

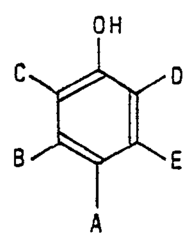

(I)

wherein A, B, C, D, and E each independently stand for hydrogen, a hydroxyl group, a substituted or unsubstituted alkyl group, a substituted or unsubstituted allyl group, a substituted or unsubstituted alkoxyl group or a substituted or unsubstituted aryl group, provided that A does not stand for a hydroxyl group and that when two or more A, B, C, D, and E each independently stand for a substituted or unsubstituted alkyl group or a substituted or unsubstituted alkoxy group, they have their respective free terminal ends or at least one of them is bonded to another group selected from said alkyl and alkoxyl groups to form a ring, with an organic halide in the presence of an alkali metal hydroxide, using as a catalyst a modified or

unmodified cyclodextrin, characterized in that said organic halide is selected from the group consisting of a carbon tetrahalide and a substituted or unsubstituted allyl halide, thereby to introduce a substituent group derived from the organic halide selected from the group consisting of a carbon tetrahalide and a substituted or unsubstituted allyl halide to the para-position of the phenol compound.

2. A process according to claim 1, wherein said substituted or unsubstituted alkyl group, said substituted or unsubstituted allyl group, said substituted or unsubstituted alkoxyl group and said substituted or unsubstituted aryl group each have carbon atoms of not more than 6 with respect to B, C, D and E and each have carbon atoms of not more than 12 with respect to A.

3. A process according to claim 1, wherein said alkali metal hydroxide is sodium hydroxide or potassium hydroxide.

4. A process according to claim 1, wherein the reaction is effected in an aqueous medium.

5. A process according to claim 1, wherein A in the formula (I) is hydrogen and said para-substituted phenol derivative is represented by the formula (II)

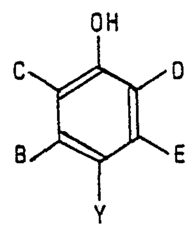

(II)

wherein B, C, D and E are as defined above and Y stands for a substituted or unsubstituted allyl group or a carboxyl group, or A in the formula (I) is a substituted or unsubstituted alkyl group, a substituted or unsubstituted allyl group, a substituted or unsubstituted alkoxyl group or a substituted or unsubstituted aryl group and said para-substituted phenol derivative is represented by the formula (III)

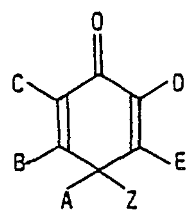

(III)

wherein A, B, C, D and E are as definede above, provided that A does not stand for hydrogen, and Z stands for a substituted or unsubstituted allyl group.

**Patentansprüche**

1. Verfahren zur Herstellung eines para-substituierten Phenolderivats, bei dem ein durch die Formel (I) dargestelltes Phenol

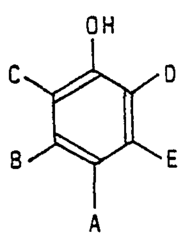

(I)

in der A, B, C, D und E jeweils unabhängig voneinader für Wasserstoff, eine Hydroxylgruppe, eine substituierte oder unsubstituierte Alkylgruppe, eine substituierte oder unsubstituierte Allylgruppe, eine substituierte oder unsubstituierte Alkoxygruppe oder eine substituierte oder unsubstituierte Arylgruppe stehen, unter der Bedingung, daß A nicht für eine Hydroxylgruppe steht, und daß dann, wenn zwei oder mehr der Substituenten A, B, C, D und E jeweils unabhängig voneinander für eine substituierte oder unsubstituierte Alkylgruppe oder eine substituierte oder unsubstituierte Alkoxygruppe stehen, diese entweder freie Enden haben oder mindestens eine unter ihnen unter Ringbildung mit einer anderen Alkyl- oder Alkoxygruppe verbunden ist, mit einem organischen Halogenid in Gegenwart eines Alkalimetallhydroxids und unter Verwendung eines modifizierten oder unmodifizierten Cyclodextrins als Katalysator umgesetzt wird, dadurch gekennzeichnet, daß das organische Halogenid aus der Tetrahalogenkohlenstoffe und substituierte oder unsubstituierte Allylhalogenide umfassenden Gruppe ausgewählt wird, wobei eine

Substituentengruppe in die para-Stellung des Phenols eingeführt wird, die sich von dem organischen Halogenid ableitet, das aus der Tetrahalogenkohlenstoffe und substituierte oder unsubstituierte Allylhalogenide umfassenden Gruppe ausgewählt ist.

2. Verfahren nach Anspruch 1, bei dem die substituierte oder unsubstituierte Alkylgruppe, die substituierte oder unsubstituierte Allylgruppe, die substituierte oder unsubstituierte Alkoxygruppe und die substituierte oder unsubstituierte Arylgruppe jeweils nicht mehr als 6 Kohlenstoffatome aufweisen, wenn sie für B, C, D und E stehen und jeweils nicht mehr als 12 Kohlenstoffatome im Fall von A besiizen.

3. Verfahren nach Anspruch 1, bei dem das Alkalimetallhydroxid Natriumhydroxid oder Kaliumhydroxid ist.

4. Verfahren nach Anspruch 1, bei dem die Reaktion in einem wäßrigen Medium durchgeführt wird.

5. Verfahren nach Anspruch 1, bei dem A in Formel (I) Wasserstoff bedeutet und das para-substituierte Phenolderivat durch die Formel (II) dargestellt wird

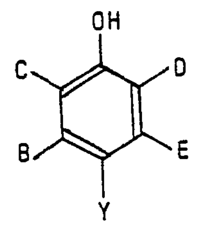

(II)

worin B, C, D und E die vorstehende Definition haben und Y für eine substituierte oder unsubstituierte Allylgruppe oder Carboxylgruppe steht, oder bei dem A in Formel (I) eine substituierte oder unsubstituierte Alkylgruppe, eine substituierte oder unsubstituierte Allylgruppe, eine substituierte oder unsubstituierte Alkoxygruppe oder eine substituierte oder unsubstituierte Arylgruppe bedeutet und das para-substituierte Phenolderivat durch die Formel (III) dargestellt wird

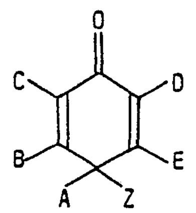

(III)

in der A, B, C, D und E die vorstehende Definition haben, vorausgesetzt daß A nicht für Wasserstoff steht, und Z für eine substituierte oder unsubstituierte Allylgruppe steht.

**Revendications**

1. Procédé de production d'un dérivé de phénol para-substitué qui comprend la réaction d'un composé de phénol représenté par la formule (I)

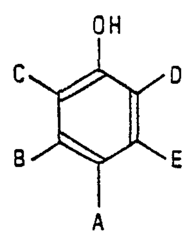

(I)

dans laquelle chacun de A, B, C, D et E indique indépendamment de l'hydrogène, un groupe hydroxyle, un groupe alcoyle substitué ou non substitué, un groupe allyle substitué ou non substitué, un groupé alcoxyle substitué ou non substitué ou un groupe aryle substitué ou non substitué, à condition que A n'indique pas un groupe hydroxyle et que lorsque deux de A, B, C, D et E, ou plus, indiquent indépendamment un groupe alcoyle substitué ou non substitué ou un groupe alcoxyle substitué ou non substitué, ils aient leurs extrémités terminales libres respectives ou au moins l'une d'entre elles liées à un autre groupe choisi parmi lesdits groupes alcoyle et alcoxyle pour former un noyau, avec un halogénure organique en présence d'un hydroxyde d'un métal alcalin, en utilisant comme catalyseur une cyclodextrine ou non modifiée, caractérisé en ce que ledit halogénure organique est choisi dans le groupe consistant en un tétrahalogénure de carbone et un halogénure d'allyle substitué ou non substitué, pour ainsi introduire un groupe substituant dérivé de l'halogénure organique choisi dans le groupe consistant en un tétrahalogénure de carbone et un halogénure d'allyle substitué ou non substitué à la position para du composé de phénol.

2. Procédé selon la revendication 1, où ledit groupe alcoyle substitué ou non substitué, ledit groupe allyle substitué ou non substitué, ledit groupe alcoxyle substitué ou non substitué et ledit groupe aryle substitué ou non substitué ont chacun des atomes de carbone, à raison de pas plus de 6 rapport à B, C, D et E et chacun a des atomes de carbone de pas plus de 12 par rapport à A.

3. Procédé selon la revendication 1, où ledit hydroxyde d'un metal alcalin est de la soude de la potasse.

4. Procédé selon la revendication 1, où la réaction est effectuée dans un milieu aqueux.

5. Procédé selon la revendication 1, où A dans la formule (I) est de l'hydrogène et ledit dérivé de phénol para-substitué est représenté par la formule (II)

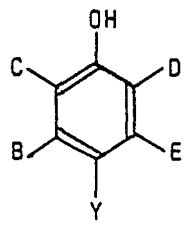

(II)

dans laquelle B, C, D et E sont tels que définis ci-dessus et Y indique un groupe allyle substitué ou non substitué ou un groupe carboxyle, ou bien A, dans la formule (I), est un groupe alcoyle substitué ou non substitué, un groupe allyle substitué ou non substitué, un groupe alcoxyle substitué ou non substitué ou un groupe aryle substitué ou non substitué et ledit dérivé de phénol para-substitué est représenté par la formule (III)

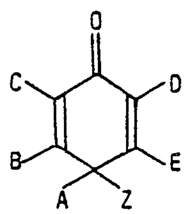

(III)

dans laquelle A, B, C, D et E sont tels que définis ci-dessus, à condition que A n'indique pas de l'hydrogène et que Z indique un groupe allyle substitué ou non substitué.